# EUROPEAN PATENT SPECIFICATION

(11) **EP 2 234 577 B1**
(45) Date of publication and mention of the grant of the patent: **23.09.2015**
(21) Application number: 09706135.2
(22) Date of filing: 23.01.2009
(51) Int. Cl.: A61K 8/02, A61K 8/891, A61K 8/898, A61Q 19/00

(54) **MOISTURIZING LIQUID LINER FOR BARRIER LAYER**
BEFEUCHTUNG EINER FLÜSSIGAUSKLEIDUNG FÜR EINE SPERRSCHICHT
REVETEMENT LIQUIDE HYDRATANT POUR COUCHE BARRIERE

(30) Priority: 30.01.2008 US 22545
(43) Date of publication of application: 06.10.2010
(73) Proprietor: Deb IP Limited, Denby, Derbyshire DE5 8JZ (GB)
(72) Inventor: HOWE, Anna, Moseley Virginia 23120 (US); ADKINS, Dana, Quinton Virginia 23141 (US); WENZEL, Scott W., Neenah Wisconsin 54956 (US); KOENIG, David William, Menasha Wisconsin 54952 (US); HOFFMAN, Douglas Robert, Greenville Wisconsin 54942 (US)
(74) Representative: Elsy, David
(86) International application number: PCT/EP2009/050746
(87) International publication number: WO 2009/095351

(56) References cited:
- EP-A1- 2 000 124
- WO-A1-03/035765
- WO-A1-2007/142629
- WO-A2-2005/036996
- US-A- 5 196 499
- US-A1- 2004 122 382
- US-A1- 2004 126 604
- Anonym: "ABIL WAX Kosmetrische Öle / Wachse für Hautpflegeprodukte - Produkt Datenblatt" Franken Chemie May 2003 (2003-05), pages 1-6, XP002594446 Retrieved from the Internet: URL:http://www.frankenchemie.de/img/DS_ABI L_Wax_d.pdf [retrieved on 2010-07-26]
- GASPERLIN M ET AL: "The structure elucidation of semisolid w/o emulsion systems", INTERNATIONAL JOURNAL OF PHARMACEUTICS, ELSEVIER BV, NL, vol. 107, no. 1, 20 June 1994 (1994-06-20) , pages 51-56, XP025554408, ISSN: 0378-5173, DOI: DOI:10.1016/0378-5173(94)90301-8 [retrieved on 1994-06-20]

## Description

### Field of the Invention:

The present invention is directed to a moisturizing liquid liner for a barrier layer.

### Discussion of the Background:

Elastomeric articles which are used in such a manner so as to contact the wearer's skin are well known. Articles such as medical gloves, for example, are anticipated to be worn by the user for prolonged periods of time. Because certain elastomeric articles are used with relatively higher frequency as well as with prolonged duration, important characteristics of such articles include their physical properties and their comfort of use.

A variety of medical gloves, e.g., surgical gloves and examination gloves, are well known and readily available in the medical field. The chemical and physical properties of elastomers used in such gloves have been researched, and gloves exhibiting desirable properties in accordance with their usage have been developed. Properties such as tensile strength as elongation modulus, as well as coatings and lubricants, which enhance their usage and/or donning characteristics, have been investigated. A variety of elastomeric polymer compositions have been examined as well, including formulations using natural and synthetic latex.

When gloves are worn for extended periods of time, body heat is generated by the hand and heavy perspiration that can cause overhydration damaging the natural skin protection afforded by the stratum corneum. After the gloves are removed from the hand and the sweat evaporates, the skin of the hand can become dry, sensitive and sometimes, infected. Such undesirable skin conditions can lead to even more serious skin problems as a loss of the epidemical lipid barrier which retains skin moisture.

In addition, those who utilize elastomeric articles, such as gloves, often work in clinical settings that require frequent hand cleaning. For example, healthcare personnel must wash their hands or at least wipe their hands with sanitary alcohol formulations many times a day. This constant cleaning may be harsh on the skin, causing excessive skin dryness that may exacerbate skin problems resulting from frequent glove use.

Pre-donning skin lotions have been developed for application to the user's skin prior to donning gloves. Such lotions are typically applied separately to the skin, and the glove is then donned afterward. Other lotions are applied to the skin after the glove has been removed. Therapeutic skin-moisturizing gloves containing water-activatable material on a skin-contacting surface are described in Berry U.S. Pat. No. 5,869,072. The water-activatable material disclosed in this reference includes polyvinyl alcohol, as well as additional ingredients such as moisturizers and vitamins and is applied onto a flexible porous sheet which is associated with a glove.

One problem associated with many lotions or creams is the deterioration of glove performance as a result of adverse effects on barrier and physical properties of the elastomer. Another problem associated with pre-coated gloves is their ability to withstand sterilization treatment and/or elevated thermal environments, encountered during the manufacturing process and storage, without adverse impact on either the coating, elastomer properties, or both. Yet another problem with such lotions or creams is the use of oily emollients, which can produce an uncomfortable greasy feeling.

Accordingly, there is a need in the field of skin-contacting elastomeric articles for improvements in their comfort to the user. Particularly advantageous would be the development of an elastomeric glove which is pre-coated with a liner layer composition which is thermally stable. Even more desirable would be such a coating layer which provides a non-greasy, comfortable feeling to the skin and moisturizing effect.

Wang et al. U.S. 2004/0126604 relates to a therapeutic, moisturizing coating composition for elastomeric articles which is applied directly onto the skin-contacting surface of the article as part of the manufacturing process.

Chou U.S. 6,630,152 and U.S. 6,425,328 and U.S. 6,274,154 describe a protective glove includes a coating of dehydrated material on its inside surface. The dehydrated material, in contact with perspiration from a hand wearing the glove, soothes the hand. Some methods of placing the coating onto the inside surface of the glove include spraying or dipping with a solution that includes Aloe Vera.

Chou U.S. 6,953,582 describes a protective glove includes a coating of dehydrated material on its inside surface. The dehydrated material, in contact with perspiration from a hand wearing the glove, soothes the hand. Some methods of placing the coating onto the inside surface of the glove include spraying or dipping with a solution that includes Aloe Vera.

Eng et al. U.S. 2006/0070167 describes a hand-friendly rubber glove article comprising a dried coating of an emulsified hand-friendly mixture comprising at least one water-soluble humectant moisturizer, at least one water-soluble lubricant, at least one water-soluble surfactant, and at least one water-insoluble occlusive moisturizer, which is finely and substantially uniformly dispersed within the mixture, which is transferred to the skin of a wearer upon activation with skin-generated moisture, and, optionally, a fabric-adherent cuff region and/or a texturized surface and methods of making the emulsified hand-friendly mixture and the glove article.

Williams U.S. 2005/0081278 describes a disposable glove which comprises a polymeric material having an inside surface for contacting the skin of a wearer. The glove further comprises a coating formed on the inside surface thereof. This coating comprises a dried coating comprising a film-forming compound and an oil-based emollient. A method of making the glove is also disclosed.

DeFina U.S. 5,614,202 describes a moisturizing glove in which a middle layer is saturated with lotion, an exterior layer of non-porous material is formed to the top side of the middle layer, and an inner layer having a plurality of pores, formed to the bottom side of the middle layer create a cavity for receiving and enveloping a human extremity, particularly a human hand.

Loo et al. U.S. 2004/0115250 describes a glove wherein the interior surfaces of the glove contain a film which includes water, glycerol and a botanical extract.

Johnson et al. US 2004/0122382 discloses an elastomeric article, such as an elastomeric glove, for example, that includes a coating on the skin-contacting surface of the article. The coating includes a carrier which may separate from the article at expected use conditions and may help to lubricate the skin. The coating also includes an additive which may provide a clinical benefit to the skin. The additive may be an emollient, a humectant, an anti-oxidant, or some other clinically beneficial additive.

Yu et al. WO 03022962 provides for a coating having microcapsules for use with a glove. The coating improves both wet and dry and donnability of the glove. The coating comprises microcapsules, water and a polyurethane for application to a glove.

Amdur WO 2004043179 describes a disposable examination glove is made by forming a disposable glove from a flexible material; coating the interior surface of the glove with a liquid carrier, Aloe Vera, and at least one α-hydroxy acid; and removing liquid carrier from the coating to form a substantially dry coating of Aloe Vera and the at least one α-hydroxy acid on the interior surface of the glove. The dry coating is bonded to the interior surface of the glove so that the coating contacts the hand of a person wearing the glove.

Amdur WO 2004043235 describes a disposable examination glove is made by forming a disposable glove from a flexible material; coating the interior surface of the glove with a liquid carrier, Aloe Vera, and allantoin; and removing liquid carrier from the coating to form a substantially dry coating of Aloe Vera and allantoin on the interior surface of the glove. The dry coating is bonded to the interior surface of the glove so that the coating contacts the hand of a person wearing the glove.

Buchalter U.S. 3,896,807 describes an article such as an article of apparel or a cream applicator which is impregnated with the oil phase of a cream formulation, in the form of a non-oily non-tacky solid, and which upon the addition of water or moisture thereto forms a therapeutic skin cream.

Accordingly, a moisturizing liner layer for a barrier layer is sought.

### SUMMARY OF THE INVENTION:

Accordingly, one object of the present invention is to provide a moisturizing liquid liner for a barrier layer comprising a specific hydrophobic moiety, an emulsifier and the cationic moiety Quaternium-80.

According to a second embodiment of the present invention is to provide a moisturizing liquid liner for a barrier layer comprising a specific hydrophobic moiety, an emulsifier, the cationic moiety Quaternium-80 and water.

The present invention describes a method of moisturizing skin by contacting skin with an elastomeric article comprising on the skin-contacting surface of a barrier layer, a moisturizing liquid liner for said barrier layer comprising a hydrophobic moiety.

The present invention describes a substrate comprising a barrier layer and on the skin-contacting surface a moisturizing liquid liner for said barrier layer comprising a hydrophobic moiety and an emulsifier.

These and other objects of the present invention have been made possible by the discovery that a combination of hydrophobic moiety and emulsifier provides a good liner layer function to a moisture impermeable barrier layer.

### DETAILED DESCRIPTION OF THE INVENTION:

The present invention relates to a moisturizing liquid liner comprising a specific hydrophobic moiety and an emulsifier and the cationic moiety Quaternium-80 and optionally water.

Hydrophobic moiety according to the present invention must comprise a silicone oil in an amount which is at least 30 wt. %, more preferably at least 40 wt.% even more preferably at least 50 wt. % of the hydrophobic moiety. The silicone oil will be a liquid oil have a silicon backbone which is a C₁₋₂₂ dialkyl polysiloxane such as a dimethicone. Further the backbone has grafted onto it C₄₋₂₂ alkyl groups, more preferably cetyl groups. The silicone oil will preferably have a molecular weight ranging from 700 to 20,000, more preferably 800 to 14,000, more preferably 900 to 1,000, more preferably about 1,000. The siloxane backbone of the silicone oil is not particularly limited and may range from about 5 to 100 siloxane units.

In addition to the silicone oil, the hydrophobic moiety may further comprise a hydrophobic oil which is miscible with the silicone oil, such as hydrocarbon oils, dialkyl ethers, alkyl carboxylates, alkoxylates ethers and esters.

Non-limiting examples of suitable hydrophobic oils include PPG-15 stearyl ether, dicaprylylether, diethylhexyl carbonate, C₁₂₋₁₅ alkyl benzoate, ethylhexyl palmitate, hexyldecyl stearate, isocetyl palmitate, isopropyl laurate, isopropyl palmitate, caprylic/capric triglyceride, triisostearin, propylene glycol myristyl ether, bis-PEG/PPG-20/20 dimethicone, PEG/PPG-4/12 dimethicone, cetyl palmitate, stearyl palmitate, cetyl stearate, isopropyl laurate, isopropyl myristate, isopropyl palmitate, caprylic/capric triglyceride, and combinations thereof. Ethers such as eucalyptol, ceteraryl glucoside, dimethyl isosorbic polyglyceryl-3 cetyl ether, polyglyceryl-3 decyltetradecanol and propylene glycol myristyl ether.

Suitable silicone oils are well known to those of ordinary skill in the art such as comb-like modified siloxanes such as ABIL^{®} EM90, ABIL^{®} Wax 9801, ABIL^{®} Wax 9814, and ABIL^{®} Wax 9840, products of Evonik Degussa, alkyl dimethicones, alkyl methicones, alkyl dimethicone copolyols, phenyl silicones, alkyl trimethylsilanes, dimethicone, dimethicone crosspolymers, cyclomethicone, and combinations thereof. Preferably the hydrophobic moiety is a cetyl dimethicone, such as ABIL^{®} Wax 9801.

The amount of hydrophobic moiety in the moisturizing liner composition is not particularly limited and is typically present in an amount of from 20-70 wt. %, more preferably 40-60 wt. %, even more preferably about 50 wt. % based on the weight of the liner composition.

The hydrophobic moiety may further comprise an auxiliary silicone oil such as cyclomethicone, in amounts up to 50 wt.%, preferably from 30 to 46 wt. % based on the total weight of the composition, in order to reduce the viscosity of the moisturizing liquid liner layer. A reduced viscosity allows the liquid liner layer to display enhanced spreadability providing for easier application to the surface of a barrier layer.

Emulsifiers according to present invention are not particularly limited and will preferably have a hydrophilic/lipophilic balance HLB from 3 to 8 and behave as water-in-oil emulsifiers. Any suitable emulsifier may be included in the skin benefit compositions of the present disclosure including carbon based emulsifiers, silicon based emulsifiers, non-ionic emulsifiers, cationic emulsifiers and combinations thereof. Suitable carbon based emulsifiers include carbon based emulsifies such as Polyglyceryl-4 Isostearate. Silicon based water-in-oil emulsifiers include alkylene oxide graft modified silicone oils. Suitable alkylene oxides include ethylene oxide, propylene oxide, butylenes oxide and mixtures thereof. Grafting of alkylene oxide groups may occur randomly or in blocks. Preferably the grafting is block grafting of ethylene oxide and propylene oxide in a weight ratio of 10 to 1. Other emulsifiers include Bis-PEG/PPG-14/14 dimethicone (ABIL^{®}EM 97 a product of Evonik), polyglyceryl-3 oleate (ISOLAN^{®}GO 33 a product of Goldschmidt), polyglyceryl-4 diisostearte/polyhydrosysteate/sebacate (ISOLAN^{®}GPS a product of Goldschmidt), polyglyceryl-2 dipolyhydroxystearate, polyglyceryl-3 polyrincinoleate, PEG-30 dipolyhydroxystearate, glyceryl stearate, hydrogenated vegetable glycerides phosphate, sorbitan oleate, sorbitan sesquioleate, sorbitan isostearate, sorbitan trioleate, polyglyceryl-3-diisostearate, polyglyceryl-4 oleate or combinations thereof.

The emulsifier may be used alone, or in combination with other emulsifiers. Preferably the emulsifier is a mixture of polyglyceryl-4 isostearate, cetyl PEG/PPG-10/1 Dimethicone, Hexyl Laurate sold under the trademark ABIL^{®} WE 09 a product of Evonik.

The amount of emulsifier in the moisturizing liner composition is not particularly limited and is typically present in an amount of from 0.4 to 10 wt. %, preferably 1 to 8 wt. %, more preferably from 3 to 6 wt. %, more preferably 4 to 5 wt. % based on the weight of the liner composition.

In use, the hydrophobic moiety in conjunction with the emulsifier form an occlusive layer on the skin.

Suitable cationic moieties according to the present invention are not particularly limited and are typically those used as fabric softeners.

Hydrocarbon fabric softeners suitable for use herein are selected from the following classes of compounds:
Cationic quaternary ammonium salts. The counter ion of such cationic quaternary ammonium salts may be a halide, such as chloride or bromide, methyl sulphate, or other ions well known in the literature. Preferably the counter ion is methyl sulfate or any alkyl sulfate or any halide, methyl sulfate being most preferred.

Examples of cationic quaternary ammonium salts include conventionally known monoalkyl quaternary ammonium salts, dialkyl quaternary ammonium salts, and tetra-alkyl quaternary ammonium salts, but are not limited to:
(1) Acyclic quaternary ammonium salts having at least two C₈ to C₃₀, preferably C₁₂ to C₂₂ alkyl or alkenyl chains, such as: Dimethyl Ditallow Ammonium Methylsulfate, di(hydrogenated tallow)dimethyl ammonium methylsulfate, distearyldimethyl ammonium methylsulfate, dicocodimethyl ammonium methylsulfate and the like. It is especially preferred if the cationic moiety is a water insoluble quaternary ammonium material which comprises a compound having two C₁₂ to C₁₈ alkyl or alkenyl groups connected to the molecule via at least one ester link. It is more preferred if the quaternary ammonium material has two ester links present. An especially preferred ester-linked quaternary ammonium material for use in the invention can be represented by the formula: 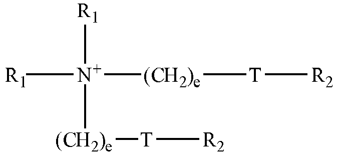 wherein each R₁ group is independently selected from C₁ to C₄ alkyl, hydroxyalkyl or C₂ to C₄ alkenyl groups; T is either 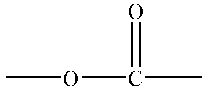 or 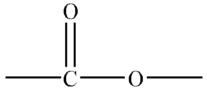 and wherein each R₂ group is independently selected from C₈ to C₂₈ alkyl or alkenyl groups; and e is an integer from 0 to 5.
A second preferred type of quaternary ammonium material can be represented by the formula: 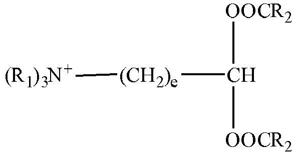 wherein R₁, e and R₂ are as defined above.
(2) Cyclic quaternary ammonium salts of the imidazolinium type such as di(hydrogenated tallow)dimethyl imidazolinium methylsulfate, 1-ethylene-bis(2-tallow-1-methyl) imidazolinium methylsulfate and the like;
(3) Diamido quaternary ammonium salts such as: methyl-bis(hydrogenated tallow amidoethyl)-2-hydroxethyl ammonium methyl sulfate, methyl bi(tallowamidoethyl)-2-hydroxypropyl ammonium methylsulfate and the like;
(4) Biodegradable quaternary ammonium salts such as N,N-di(tallowoyl-oxy-ethyl)-N,N-dimethyl ammonium methyl sulfate and N,N-di(tallowoyl-oxy-propyl)-N,N-dimethyl ammonium methyl sulfate. Biodegradable quaternary ammonium salts are described, for example, in U.S. Pat. Nos. 4,137,180, 4,767,547 and 4,789,491.

Preferred biodegradable quaternary ammonium salts include the biodegradable cationic diester compounds as described in U.S. Pat. No. 4,137,180.

These cationic moieties are more definitively described in U.S. Pat. No. 4,134,838.

In addition, the cationic moiety may be a cationically modified silicone based surfactant, which are well known to those of ordinary skill in the art and exemplified by silicone bisquaternary compounds. Suitable bisquaternary compounds may have a siloxane backbone of 10 to 100, preferably 60-100 siloxane units, which are terminally modified with quaternary ammonium groups at each end. In one embodiment the terminal ends of the bisquaternary compound is comprised of alkyl amide quaternary ammonium hydroxy groups such as those resulting from ring opening of an epoxide with an alkyl amide amine. At least one cationic moiety of the moisturizing liquid liner composition is a silicone bisquaternary is quaternium-80, such as ABIL^{®} Quat 3474, a product of Evonik.

To the extent that the components of the moisturizing liquid liner layer comprise salt forming groups, such as the silicone bisquaternary, the moisturizing liquid liner layer may be comprised of salts of such salt forming groups. Suitable salts are known to those of ordinary skill in the art and would include acetate salts of quaternary ammonium groups.

The amount of cationic moiety in the moisturizing liquid liner composition is not particularly limited and is typically present in an amount of from 0.01 to 10 wt. %, preferably 0.5 to 8 wt. %, more preferably about 1 to 5 wt. %, even more preferably about 1 wt. % based on the weight of the liner composition.

The moisturizing liquid liner layer may further comprise components which do not adversely affect the composition such as an emollient such as petrolatum, mineral oil, vegetable based oils, squalane, eiethylhexylcyclohexane, phenyl silicones and alkyl trimethylsilanes.

In one embodiment, the skin benefit agent may be held in the skin benefit composition in liposomes. A liposome is a vehicle for delivering agents to the skin. More specifically, a liposome is a microscopic sphere formed from a fatty compound, a lipid, surrounding a water-based agent, such as a moisturizer or an emollient. When the liposome is rubbed into the skin, it releases the agent throughout the stratum corneum.

In another embodiment, the beneficial agent may be present in the carrier in the form of a microencapsulant. A microencapsulant is a sphere of an emollient surrounded by a gelatin membrane that prevents the emollient from reacting with other ingredients in the composition and helps distribute the emollient more evenly when pressure is applied and the membrane is broken. The process of forming these beads is called microencapsulation and is generally known in the art.

In one embodiment, the moisturizing liquid liner layer does not comprise an α hydroxyl lactone. In another embodiment, the moisturizing liquid liner layer does not comprise a polyhydric alcohol. In another embodiment, the moisturizing liquid liner layer does not comprise a self-emulsifying wax as described in Johnson et al. US 2004/0122382. In another embodiment, the moisturizing liquid liner layer does not comprise a mixture of a fatty alcohol, a fatty acid and or a fatty ester with a C₂₀ surfactant. In another embodiment, the moisturizing liquid liner layer does not comprise any of a cellulose, collagen and or vinylpyrolidone derived cationic polymer. In another embodiment, the moisturizing liquid liner layer does not comprise a silicone wax.

As a result of use as a liner layer the moisturizing liquid liner composition will typically absorb moisture from skin, in amount of up to 90 wt.%. Typically the moisturizing liquid liner layer will contain less than 70 wt. % of water. In one embodiment, the moisturizing liquid liner composition is anhydrous. When the moisturizing liquid liner composition comprises an emulsifier, the water is dispersed within the liner composition as an emulsion. Typically, prior to use, the liner layer will comprise only a small amount of water (e.g. <5 wt. %, more preferably < 1 wt. %, even more preferably < 0.5 wt. %), if any water at all, as water will tend to increase the viscosity of the liner layer.

The composition according to the present invention may be manufactured by simply mixing the components, until a homogeneous mixture is obtained.

The moisturizing liquid liner layer is deposited on a surface of a moisture impermeable barrier layer to form a substrate. Suitable barrier layers may be any natural or synthetic elastomeric polymer which is chemically compatible with the coating composition ingredients. Suitable elastomers include, but are not limited to, synthetic and natural rubber latex. Natural rubber that can be used includes rubber made from hevea rubber latex and guayule rubber latex. Synthetic rubber polymers which can be used include nitrile rubber, nitrile butadiene rubber, polyurethane, polyisoprene, neoprene,polychloroprene, styrene block co-polymers and polymer blends thereof. Synthetic rubbers that can be used also include acrylic diene block co-polymer, acrylic rubber, butyl rubber, EPDM rubber, polybutadiene, chlorosulfonated polyethylene rubber and fluororubber. Elastomers may be used alone or in combination as mixtures. In a preferred embodiment, the elastomer is a nitrile butadiene rubber, latex, vinyl and nitrile rubber. The surface of the barrier layer, may itself be porous provided that the barrier layer prevents moisture from passing through the barrier layer.

Moisture impermeable barrier layers may be prepared by conventional methods known to those of ordinary skill in the art without undue experimentation, such as techniques used in the manufacture medical gloves.

The liner layer composition of the invention can further contain additional beneficial ingredients provided such are chemically compatible with the composition of the invention and do not adversely affect the desired therapeutic properties of the composition. Additional ingredients that can be included in the coating composition include, but are not limited to, moisturizers, antimicrobial agents, antiinflammatory agents, topical cleansing agents, anti-perspiration agents, skin conditioning agents, medicants, sunscreens, anti-aging agents, and the like. Dimethicone (350 cs) in amounts of from 0.5 to 10 wt. % based on the liner layer composition is a suitable moisturizer.

The moisture impermeable barrier layer is typically incorporated into an article, which may be elastomeric, such as a hand glove, a glove liner, a surgical mask for medical use, socks, a spa masque, a condom or an article of clothing such as a shirt, pants or undergarments, in which a surface thereof comes in contact with skin. The barrier layer may also be incorporated into the skin contacting surface of a prosthetic liner for receiving a residual limb. In one embodiment, the article is not a hand glove. The surface of the article which is to come into contact with skin is provided with a layer of the moisturizing liquid barrier layer of the present invention. The composition may also be applied directly onto the skin, as a liquid by rubbing or by spraying on the hands and/or other body parts.

The invention provides a moisturizing liquid liner composition to be applied to a skin-contacting surface of a skin-contacting barrier layer. Then the barrier layer is part of an article which is worn, the moisturizing liquid liner composition hydrates upon moisture contact and transfers onto the wearer's skin during use, providing the topical benefits afforded by the ingredients of the composition. The moisture from the wearer's skin converts the liner layer composition into a hydrated, liquid "lotion" form, and the "lotion" form is transferred directly from the skin-contacting surface of the barrier layer onto the wearer's skin while worn. The moisturizing liquid liner composition continues to provide prolonged therapeutic benefit to the skin following its removal. The liner layer composition is chemically compatible with the material of the barrier layer, and has no substantial impact on the physical properties of the barrier layer. The liner layer composition of the invention is thermally stable and survives elevated temperatures associated with manufacturing and certain sterilization treatments. The liner layer composition has a pleasant non-sticky, non-greasy feel.

The moisturizing liquid liner layer composition is typically deposited on the surface of the barrier layer in an amount effective to provide a moisturizing effect. Non-limiting amount preferably include amounts of 0.01 to 10 mg/cm² of barrier layer, preferably 0.1 to 1 mg/cm² of barrier layer, more preferably 0.3 to 0.6 mg/cm² of barrier layer, more preferably about 0.5 mg/cm² of barrier layer.

A further advantage of the claimed moisturizing liquid liner layer is that after use, there is a reduced odor which is typically associated with barrier layer use.

By contacting the skin with a barrier layer containing a moisturizing liquid liner layer, a method of moisturizing the skin is realized. The contact of the wearer's skin with the skin benefit composition will produce sufficient moisture and heat which may cause emulsification of at least a portion of the skin benefit composition, and formation of a lotion-like emulsion that has moisturizing and other skin health benefit effects on the skin of the article's wearer. The beneficial effects of the liner layer composition can be felt both during article wear, after the article, such as a glove, is removed, and after subsequent washing of the skin. Advantageously, the liner layer composition's the skin benefit may occur even after relatively short glove wear times, for example, of about 1 minute. The articles of the present disclosure are thus particularly useful in healthcare and food preparation settings where frequent hand washing oftentimes causes skin irritation and drying.

Having generally described this invention, a further understanding can be obtained by reference to certain specific examples which are provided herein for purposes of illustration only and are not intended to be limiting unless otherwise specified.

### Brief description of the drawings:

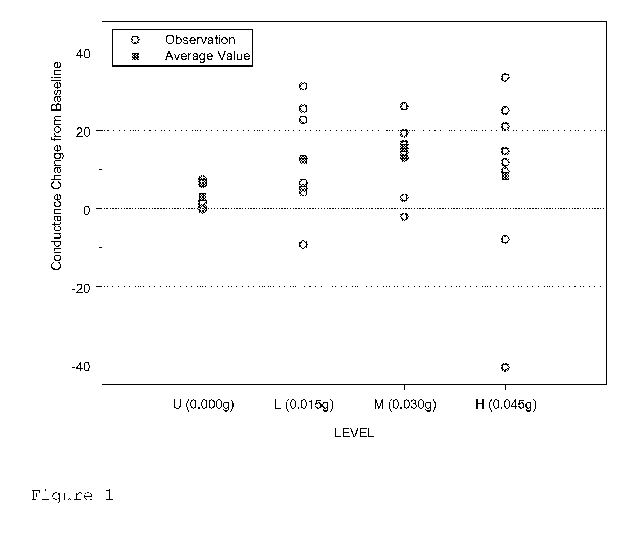

Figure 1 shows the results of conductance measurements in the panel test of example 2.

### EXAMPLES:

### Example 1:

### Formulation A

| | |
|---|---|
| ABIL^{®} WE 0 9 | 4.0 % |
| Cyclomethicone | 46.0 % |
| ABIL^{®} Was 9801 | 47 % |
| ABIL^{®} Quat 3474 | 1.0 |
| Dimethicone (350 cst) | 2.0% |

The formulation was prepared by mixing each material at room temperature until dispersed.

### Example 2:

In this example, the skin benefit composition prepared in Example 1 was evaluated for its effect on skin moisturization when used in combination with a glove.

Sixteen (16) subjects between the ages of 18 and 68 were recruited for this study. Individuals with abnormal skin pigmentation at the test sites, skin disease, skin damage, skin damage due to sun exposure, tattoos or bruises on the testing areas of the arms, or excessive dryness or erythema were excluded. The subjects were instructed not to use skin creams, oils, ointment, powders, perfumes, or lotions on the hands less than 24 hours prior to and during testing.

On the day of testing, the subjects were acclimated to a temperature and humidity controlled room (70° ± 2°F; 40% ± 5% relative humidity) for 15 minutes prior to baseline testing. After equilibration, baseline measurements were taken for skin moisture (conductance).

Baseline conductance measurements were taken at test sites on the back of the hands approximately 2 cm below the knuckles using a DermaLab Moisture Flat Probe (Cortex Technology, Hadsund Denmark). Conductance is the cosmetic industry standard for measuring moisture in the skin. The DermaLab Moisture Flat Probe was used for all measurements.

It uses electrodes arranged as concentric rings to send a series of alternating electrical currents through the skin. Resistance to the currents indicates the water binding capacity of the stratum corneum, or moisture level, and provides a conductance reading. A higher conductance reading indicates a higher level of moisture in the skin. The instrument's probe was placed at the test site on the subject's hand and 5 second continuous measurements were taken in triplicate.

At the conclusion of baseline measurements, a glove was put on the hand of the subject. The gloves used in this test were nitrile gloves that were treated with either 0.015 g/g, 0.030 g/g, or 0.045 g/g add-on amount of the composition prepared in Example 1, or were controls containing no composition add-on. The gloves were worn for 10 minutes. The gloves were removed after 10 minutes and subjects were instructed to remain in the temperature/humidity controlled room for an additional 20 minutes to allow the perspiration from their hands to flash off. After this 20 minute wait, final conductance measurements were taken using the procedure described above. A comparison of conductance measurements for subjects that wore gloves having the different composition add-on amounts or untreated control gloves relative to the baseline conductance measurements were performed. The results are shown in Figure 1.

The results indicated that all treated gloves provided an increase in hand moisturization as compared to the untreated gloves. As can be seen from Figure 1, the gloves containing the three different composition add-on levels provided a greater statistically significant increase in conductance over the untreated glove. Surprising, it was observed that the glove containing the low composition add-on level (i.e., 0.015 g/g) provided an equivalent increase in hand moisturization as the glove containing the high composition add-on level (i.e., 0.045 g/g), implying that only a small quantity of composition is needed on the glove to deliver a measurable skin health benefit.

A second study was conducted to determine hand feel following wearing the treated glove. 40 healthcare professionals from 18-65 years old were recruited to participate. An untreated glove and glove treatments of 0.015 g/g and 0.045 g/g were examined. Subjects wore the gloves for 10 minutes, removed them, and then waited 15 minutes. After the 15 minute wait, subjects were asked to indicate how their hands felt.

Compared to the untreated glove, a higher percentage of subjects described their hands after wearing either treated glove as softer, smoother, less irritated, less tight, and less flaky compared to the control codes. there was little difference in the numbers between 0.015 g/g and 0.045 g/g treatment codes.

## Claims

1. A moisturizing liquid liner composition comprising:
a hydrophobic moiety;
an emulsifier and
Quaternium-80,
wherein said hydrophobic moiety is a C₁₋₂₂ dialkylpolysiloxane, with its backbone having grafted onto it further C₄₋₂₂ alkyl groups.

2. The moisturizing liquid liner composition of claim 1, wherein said emulsifier is a silicone based water-in-oil emulsifier.

3. The moisturizing liquid liner composition of any claim 1 or 2, wherein said emulsifier comprises Polyglyceryl-4- Isostearate, cetyl PEG/PPG-10/1 Dimethicone and Hexyl Laurate.

4. The moisturizing liquid liner composition of any of the claims 1 to 3, wherein said composition comprises 20 to 70 wt. % of said hydrophobic moiety.

5. The moisturizing liquid liner composition of any of the claims 1 to 4, wherein said emulsifier has a Hydrophilic-Lipophilic Balance of from 3 to 8.

6. The moisturizing liquid liner composition of any of the claims 1 to 5, wherein said emulsifier is present in an amount of 0.4 to 10 wt. % based on the weight of said composition.

7. The moisturizing liquid liner composition of any of the claims 1 to 6, wherein Quaterium-80 is present in an amount of from 0.01 to 10 wt. % based on the weight of said composition.

8. The moisturizing liquid liner composition of any of the claims 1 to 7, wherein said hydrophobic moiety further comprises cyclomethicone.

## Patentansprüche

1. Zusammensetzung für eine befeuchtende Flüssigauskleidung, umfassend:
Eine hydrophobe Komponente;
einen Emulgator und
Quaternium-80,
wobei die hydrophobe Komponente ein C1-22-Dialkylpolysiloxan ist, an dessen Rückgrad weitere C4-22-Alkylgruppen aufgepfropft sind.

2. Zusammensetzung für eine befeuchtende Flüssigauskleidung nach Anspruch 1, bei der der Emulgator ein silikonbasierter Emulgator für Wasser-in-Öl ist.

3. Zusammensetzung für eine befeuchtende Flüssigauskleidung nach Anspruch 1 oder 2, bei der der Emulgator Polyglyceryl-4 isostearat, Cetyl PEG/PPG-10/1-Dimethicon und Hexyllaurat umfasst.

4. Zusammensetzung für eine befeuchtende Flüssigauskleidung nach einem der Ansprüche 1 bis 3, bei der die Zusammensetzung 20 bis 70 Gew.-% der hydrophoben Komponente umfasst.

5. Zusammensetzung für eine befeuchtende Flüssigauskleidung nach einem der Ansprüche 1 bis 4, bei der der HLB-Wert der des Emulgators zwischen 3 und 8 beträgt.

6. Zusammensetzung für eine befeuchtende Flüssigauskleidung nach einem der Ansprüche 1 bis 5, bei der der Emulgator in einer Menge zwischen 0,4 und 10 Gew.-%, bezogen auf das Gewicht der Zusammensetzung, vorhanden ist.

7. Zusammensetzung für eine befeuchtende Flüssigauskleidung nach einem der Ansprüche 1 bis 6, bei der Quaternium-80 in einer Menge zwischen 0,01 und 10 Gew.-%, bezogen auf das Gewicht der Zusammensetzung, vorhanden ist.

8. Zusammensetzung für eine befeuchtende Flüssigauskleidung nach einem der Ansprüche 1 bis 7, bei der die hydrophobe Komponente des Weiteren Cyclomethicon umfasst.

## Revendications

1. Composition de revêtement liquide hydratant comprenant :
un groupement hydrophobe ;
un émulsifiant et
du Quaternium-80,
dans laquelle ledit groupement hydrophobe est un dialkylpolysiloxane en C₁ à C₂₂, sur le squelette duquel sont greffés des groupes alkyles en C₄ à C₂₂ supplémentaires.

2. Composition de revêtement liquide hydratant selon la revendication 1, dans laquelle ledit émulsifiant est un émulsifiant d'eau dans l'huile à base de silicone.

3. Composition de revêtement liquide hydratant selon la revendication 1 ou 2, dans laquelle ledit émulsifiant comprend du polyglycéryl-4-isostéarate, de la cétyl PEG/PPG-10/1 diméthicone et du laurate d'hexyle.

4. Composition de revêtement liquide hydratant selon l'une quelconque des revendications 1 à 3, dans laquelle ladite composition comprend de 20 à 70 % en poids dudit groupement hydrophobe.

5. Composition de revêtement liquide hydratant selon l'une quelconque des revendications 1 à 4, dans laquelle ledit émulsifiant présente un rapport hydro-lipophile de 3 à 8.

6. Composition de revêtement liquide hydratant selon l'une quelconque des revendications 1 à 5, dans laquelle ledit émulsifiant est présent en une quantité de 0,4 à 10 % en poids par rapport au poids de ladite composition.

7. Composition de revêtement liquide hydratant selon l'une quelconque des revendications 1 à 6, dans laquelle le Quaternium-80 est présent en une quantité de 0,01 à 10 % en poids par rapport au poids de ladite composition.

8. Composition de revêtement liquide hydratant selon l'une quelconque des revendications 1 à 7, dans laquelle ledit groupement hydrophobe comprend en outre de la cyclométhicone.
